# EUROPEAN PATENT APPLICATION

(11) **EP 4 782 028 A1**
(43) Date of publication of application: **29.07.2026**
(21) Application number: 23952528.0
(22) Date of filing: 19.09.2023
(51) Int. Cl.: A61M 5/142, A61M 5/145

(54) **CONTROL SYSTEM FOR BLOOD GLUCOSE MONITORING AND MANAGEMENT**

(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2023/119735
(87) International publication number: WO 2025/059863

(57) **Abstract**

The invention discloses a control system for blood glucose monitoring and management. The first working mode of the control system is configured to control the blood glucose monitoring device to perform real-time blood glucose monitoring, and the system enters the second working mode based on a trigger. The second working mode at least is configured to control the blood glucose monitoring device to perform real-time blood glucose monitoring and to control the insulin infusion device to perform insulin infusion. By the trigger, the control system transitions from the first working mode of real-time blood glucose monitoring by controlling the blood glucose monitoring device to the second working mode of at least real-time blood glucose monitoring by controlling the blood glucose monitoring device and insulin infusion by controlling the insulin infusion device, preventing patients from accidentally entering unnecessary or unacceptable working mode and improving the safety of blood glucose monitoring and management for patients.

## Description

### TECHNICAL FIELD

The present invention mainly relates to the field of medical device, and in particular to a control system for blood glucose monitoring and management.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose data through a transmitter to the remote device in real-time for the user to view. This monitoring method is called Continuous Glucose Monitoring (CGM).

Depending on the severity of the disease and the health status of the individual patient, the doctor will give different recommendations, for example, for patients with very mild symptoms, it may only be necessary to measure a small amount of fingertip blood for testing on a regular basis. Diabetic patients with mild symptoms can complete self-monitoring and management of blood glucose through continuous glucose monitoring, while diabetic patients with more severe symptoms need to be treated by the doctor, adopting targeted treatment plans to restore the blood glucose level of diabetic patients to a relatively stable state, and at the same time, providing personalized medication delivery, diet and exercise plans to facilitate the patients to achieve self-monitoring and management of blood glucose. The device used to perform continuous glucose monitoring is typically a CGM, and the device used to perform drug infusion is typically an insulin pump. Because the inaccuracy of insulin infusion will cause a risk of Hyperglycemia and hypoglycemia to patients, the doctor must evaluate that the patients are physically able to use an insulin pump before using an insulin pump for insulin infusion, and the patient also need to follow the insulin infusion solution established by the doctor.

The main production manufacturers of blood glucose monitoring and insulin infusion, such as the blood glucose monitoring equipment (CGM) manufacturers Abbott and Dexcom etc., and the insulin infusion equipment (insulin pumps) manufacturers such as the Insulet, etc., all of them use their own special handheld device (PDM) to control the corresponding CGM or insulin pumps. When CGMs and/or insulin pumps are required to be used, they need to be controlled separately by the appropriate control devices. Medtrum simultaneously produces CGM and insulin pumps, and considers the compatibility of CGM and insulin pump when producing corresponding control equipment. Therefore, a single control device can be used to simultaneously control CGM and/or insulin pump. As previously mentioned, since not all patients need to use the CGM and the insulin pump at the same time, if the user interface of the control device displays functions related to the CGM and the insulin pump at the same time, for a patient who does not need the function of the insulin pump, displaying both the functions related to the CGM and the insulin pump at the same time in the user interface not only make the interface more complicated, which affects the user's visual experience, but on the other hand, there is a possibility of affecting the normal use of the CGM due to the patient's mis-operation on the insulin pump.

Therefore, in the prior art, there is an urgent need for a control system that can realize the safe control of CGM and insulin pumps according to different situations by simple operation method.

### BRIEF SUMMARY OF THE INVENTION

The embodiment of the present invention discloses a control system for blood glucose monitoring and management. The first working mode of the control system is configured to control the blood glucose monitoring device to perform real-time blood glucose monitoring, and the system enters the second working mode based on a trigger. The second working mode at least is configured to control the blood glucose monitoring device to perform real-time blood glucose monitoring and to control the insulin infusion device to perform insulin infusion. By a trigger, the control system transitions from the first working mode of real-time blood glucose monitoring by controlling the blood glucose monitoring device to the second working mode of at least real-time blood glucose monitoring by controlling the blood glucose monitoring device and insulin infusion by controlling the insulin infusion device, preventing patients from accidentally entering unnecessary or unacceptable working mode and improving the safety of blood glucose monitoring and management for patients.

The invention discloses a control system for blood glucose monitoring and management, comprising a processor, and a memory storing programming instructions executable by the processor to cause the control system to: operate in at least a first working mode, configured to control a blood glucose monitoring device to perform real-time blood glucose monitoring; upon a trigger, transition to a second working mode configured to control the blood glucose monitoring device to perform real-time blood glucose monitoring and to control an insulin infusion device to perform insulin infusion.

According to one aspect of the present invention, the trigger at least includes a patient account that needs to enter the second working mode is included in a whitelist of a backend administrator.

According to one aspect of the present invention, the whitelist is generated by a doctor sends a request to add a whitelist to the backend administrator.

According to one aspect of the present invention, the backend administrator directly controls the control system to enter the second working mode after adding the whitelist.

According to one aspect of the present invention, the backend administrator sends a security code to the patient account after adding the whitelist, and the patient controls the control system to enter the second working mode via the security code.

According to one aspect of the present invention, a patient alerts the doctor to send the request to the backend management.

According to one aspect of the present invention, the backend administrator sends feedback to the doctor after receiving the request, the doctor notices a patient to send another request to the backend administrator upon receipt of the feedback.

According to one aspect of the present invention, the backend administrator directly controls the control system to enter the second working mode after receiving the request.

According to one aspect of the present invention, the backend administrator sends a security code to the patient account after receiving the request, and the patient controls the control system to enter the second working mode via the security code.

According to one aspect of the present invention, the second working mode further includes an auto mode.

According to one aspect of the present invention, the trigger for the auto mode at least includes a patient account that needs to enter the auto mode is included in a whitelist of a backend administrator.

According to one aspect of the present invention, the whitelist is generated by a doctor sends a request to add a whitelist to the backend administrator.

According to one aspect of the present invention, the backend administrator directly controls the control system to enter the auto mode after adding the whitelist.

According to one aspect of the present invention, the backend administrator sends a security code to the patient account after adding the whitelist, and the patient controls the control system to enter the auto mode via the security code.

According to one aspect of the present invention, a patient alerts the doctor to send the request to the backend management.

According to one aspect of the present invention, the backend administrator sends feedback to the doctor after receiving the request, the doctor notices a patient to send another request to the backend administrator upon receipt of the feedback.

According to one aspect of the present invention, the backend administrator directly controls the control system to enter the auto mode after receiving the request.

According to one aspect of the present invention, the backend administrator sends a security code to the patient account after receiving the request, and the patient controls the control system to enter the auto mode via the security code.

According to one aspect of the present invention, the control system is a smartphone.

According to one aspect of the present invention, the control system is an APP in the smartphone.

According to one aspect of the present invention, after the control system enters the second working mode, insulin infusion related functions displayed on a main interface of the APP.

According to one aspect of the present invention, the control system exits the second working mode when the patient account is removed from the whitelist.

Compared with the prior art, the technical solution of the present invention has the following advantages:
In the control system for blood glucose monitoring and management disclosed in the patent, the first working mode of the control system is used to control the blood glucose monitoring device to perform real-time blood glucose monitoring, and the system enters the second working mode based on a trigger. The second working mode at least is used to control the blood glucose monitoring device to perform real-time blood glucose monitoring and control the insulin infusion device to perform insulin infusion. By a trigger, the control system transitions from the first working mode of real-time blood glucose monitoring by controlling the blood glucose monitoring device to the second working mode of at least real-time blood glucose monitoring by controlling the blood glucose monitoring device and insulin infusion by controlling the insulin infusion device, preventing patients from accidentally entering unnecessary or unacceptable working mode and improving the safety of blood glucose monitoring and management for patients.

Furthermore, the trigger is at least based on a whitelist that exists in the backend administrator's account, further improving the security of blood glucose monitoring and management for patients.

Furthermore, the whitelist request is sent by the doctor to the backend administrator, indicating that after doctor's assessment, the patient is indeed ready to be admitted to enter into the second working mode, ensuring the safety of the patient's blood glucose monitoring and management.

Furthermore, the patient can send a request to the backend administrator to turn on the insulin pump function, the backend administrator will verify whether the patient account exists in the whitelist or not, if it exists, the insulin pump function will be turned on to ensure the safety of the patient's blood glucose monitoring and management, if it does not exist, it will send a feedback to the patient, requesting the doctor to send a whitelist request, and further the doctor will confirm whether the patient can enter into the second working mode. On one hand, it can ensure the safety of patient's blood glucose monitoring and management, on the one hand, reminding the doctor to send the whitelist application, so that the doctor will not be too busy and forget to send request to add some patient accounts to the whitelist of the backend administrator.

Furthermore, the backend administrator can directly control the control system to enter into the second working mode after adding the whitelist, which can simplify the patient's operation, or the backend administrator send a security code to the patient's account, and the patient can control the control system to enter into the second working mode through the security code, and the patient can independently decide when to enter into the second working mode according to the actual situation, which can provide user experience.

Furthermore, the second working mode further comprises an auto mode, which can be entered into by a trigger and it is not accessible to the patient at will, ensuring the safety of using the auto mode. The trigger is at least based on a whitelist in a backend administrator account, further improving the security of monitoring and managing the patient's blood glucose.

Furthermore, the whitelist request is sent by the doctor to the backend administrator, indicating that after doctor's assessment, the patient is indeed ready to be admitted to enter into the auto mode, ensuring the safety of the patient's blood glucose monitoring and management.

Furthermore, the patient can send a request to the backend administrator to turn on the auto mode function, the backend administrator will verify whether the patient account exists in the whitelist or not, if it exists, the auto mode function will be turned on to ensure the safety of the patient's blood glucose monitoring and management, if it does not exist, it will send a feedback to the patient, requesting the doctor to send a whitelist request, and further the doctor will confirm whether the patient can enter into the auto mode. On one hand, it can ensure the safety of patient's blood glucose monitoring and management, on the one hand, reminding the doctor to send the whitelist application, so that the doctor will not be too busy and forget to send request to add some patient accounts to the whitelist of the backend administrator.

Furthermore, the backend administrator can directly control the control system to enter into the auto mode after adding the whitelist, which can simplify the patient's operation, or the backend administrator send a security code to the patient's account, and the patient can control the control system to enter into the auto mode through the security code, and the patient can independently decide when to enter into the auto mode according to the actual situation, which can provide user experience.

Furthermore, the control system is the same APP in the smartphone, which is convenient for patients to use one APP to control both glucose monitoring and insulin infusion at the same time, improving convenience for patients to use.

Furthermore, the functions related to insulin infusion are displayed on the main interface of the APP only after the control system enters the second working mode from the first working mode, so that the patient can maintain the simplicity of the interface when only the first working mode is needed, at the same time prevent the patient from mis-operation to the functions related to the insulin pump that affects the normal use of the first working mode.

Furthermore, when the patient no longer needs to use the second working mode, the patient can close the second working mode by himself, or the doctor and/or the patient sends a turn off application to the backend administrator, and the backend administrator accepts the application and turns off the second working mode and removes the patient's account from the whitelist, that is the second working mode can be turned off in a variety of ways, which is convenient for the patient to choose, and improves the patient's user experience.

Furthermore, the functions related to auto mode are displayed on the interface of the APP only after the control system enters into the auto mode from the first working mode, so that the patient can maintain the simplicity of the interface when auto mode isn't needed, at the same time prevent the patient from mis-operation to the auto mode that affects the normal use of other working mode.

Furthermore, when the patient no longer needs to use the auto mode, the patient can close the auto mode by himself, or the doctor and/or the patient sends a turn off application to the backend administrator, and the backend administrator accepts the application and turns off the auto mode and removes the patient's account from the whitelist, that is the auto mode can be turned off in a variety of ways, which is convenient for the patient to choose, and improves the patient's user experience.

### BRIEF DESCRIPTION OF THE DRAWINGS

FIG.1 is a schematic diagram of the module relationship of the general closed-loop artificial pancreas insulin infusion control system according to the embodiment of the present invention.
FIG.2 is a schematic diagram of the integrated continuous glucose monitoring device according to an embodiment of the present invention.
FIG.3 is a schematic diagram of the split continuous glucose monitoring device according to an embodiment of the present invention.
FIG.4a is a schematic diagram of the integrated insulin pump according to an embodiment of the present invention.
FIG.4b is a schematic diagram of the split insulin pump according to an embodiment of the present invention.
FIG. 5a is a schematic diagram of the main interface when the control system is in a first working mode according to an embodiment of the present invention.
FIG. 5b is a schematic diagram of the main interface when the control system is in a second working mode according to an embodiment of the present invention.
FIG. 6a-FIG. 6c are schematic diagrams of different operations for the control system to turn on the insulin pump function according to embodiments of the present invention.
FIG. 7a-FIG. 7c are schematic diagrams of different operations for the control system to turn on the auto mode function according to embodiments of the present invention.
FIG. 8a and FIG. 8b show schematic diagrams of the APP interface before and after turning on the auto mode function of the control system according to an embodiment of the present invention.

### DETAILED DESCRIPTION

As mentioned above, since not all diabetic patients require both CGM to monitor blood glucose and insulin pump to infuse insulin due to their different illness severity, the current control systems individually control CGM to monitor blood glucose and insulin pump to infuse insulin separately, and there is a need for a simple control system capable of controlling CGM to monitor blood glucose and insulin pump to infuse insulin at the same time, and also conveniently switching between controlling CGM to monitor blood glucose and/or insulin pump to infuse insulin according to the illness severity of the patient.

In order to solve this problem, the present invention provides a control system for blood glucose monitoring and management. The first working mode of the control system is used to control the blood glucose monitoring device to perform real-time blood glucose monitoring, and the system enters the second working mode based on a trigger. The second working mode at least is used to control the blood glucose monitoring device to perform real-time blood glucose monitoring and control the insulin infusion device to perform insulin infusion. By a trigger, the control system transitions from the first working mode of real-time blood glucose monitoring by controlling the blood glucose monitoring device to the second working mode of at least real-time blood glucose monitoring by controlling the blood glucose monitoring device and insulin infusion by controlling the insulin infusion device, preventing patients from accidentally entering unnecessary or unacceptable working mode and improving the safety of blood glucose monitoring and management for patients.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

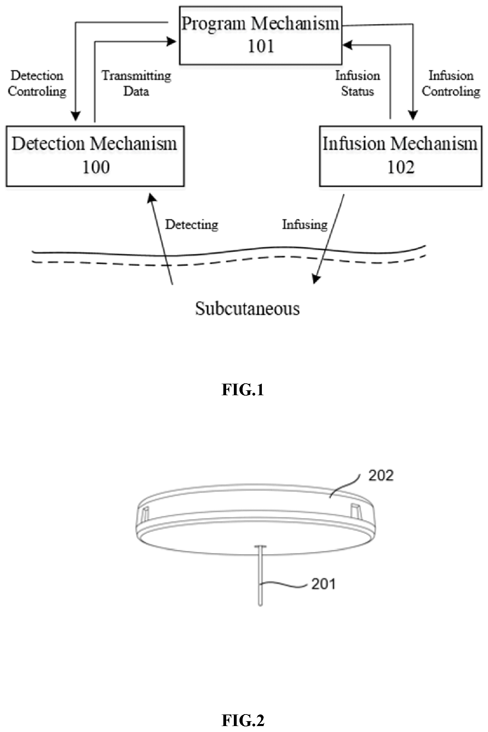

FIG.1 is a schematic diagram of the module relationship of a general closed-loop artificial pancreas insulin infusion control system according to the embodiment of the present invention.

The closed-loop artificial pancreas insulin infusion control system disclosed in the embodiment of the present invention mainly includes a detection mechanism 100, a program mechanism 101, and an infusion mechanism 102.

The detection mechanism 100 is used to continuously detect the user's real-time blood glucose (BG) level. Generally, detection mechanism 100 is a Continuous Glucose Monitoring (CGM) for detecting real-time BG, monitoring BG changes, and sending them to the program mechanism 101. The CGM includes an implantable sensor, the sensor is connected to a transmitter, the transmitter further includes a memory, a processor, a communication interface, and the like, and the transmitter is used for transmitting at least information about the blood glucose data monitored by the CGM, as well as information about an identifier of the CGM, and the like.

The infusion mechanism 102 includes the essential mechanical assemblies and electronic control unit used to infuse insulin, such as reservoir, drive structures, fluid path and infusion needles, power supplies, circuit boards, and so on, and is controlled by program mechanism 101. Generally, the infusion mechanism 102 is an insulin pump, and the electronic control unit includes a memory, a processor, a communication interface, etc. According to the current insulin infusion dose sent by program mechanism 101, infusion mechanism 102 injects the current insulin dose required into the user's body. At the same time, the real-time infusion status of infusion mechanism 102 can also be fed back to program mechanism 101.

Program mechanism 101 is used to control the detection mechanism 100 and the infusion mechanism 102. Therefore, program mechanism 101 is connected to detection mechanism 100 and infusion mechanism 102, respectively. Here, the connection refers to a conventional electrical connection or a wireless connection.

The embodiment of the present invention does not limit the specific positions and connection relationships of the detection mechanism 100, the program mechanism 101 and the infusion mechanism 102, as long as the aforementioned functional conditions can be satisfied.

As in an embodiment of the present invention, the three are electrically connected to form a single part. Therefore, the three mechanisms can be attached on only one position of the user's skin. If the three mechanisms are connected as a whole and attached in only one position, the number of the device on the user skin will be reduced, thereby reducing the interference of more attached devices on user activities. At the same time, it also effectively solves the problem of poor wireless communication between separating devices, further enhancing the user experience.

Another embodiment of the present invention is that the program mechanism 101 and the infusion mechanism 102 are electrically connected to form a single part, while the detection mechanism 100 is separately provided in another part. At this time, the detection mechanism 100 and the program mechanism 101 transmit wireless signals to realize the mutual connection. Therefore, program mechanism 101 and infusion mechanism 102 can be attached to the user's skin position while the detection mechanism 100 is attached to the other position.

Another embodiment of the present invention is that the program mechanism 101 and the detection mechanism 100 are electrically connected, forming a single part, while the infusion mechanism 102 is separately provided in another part. The infusion mechanism 102 and the program mechanism 101 transmit wireless signals to realize the mutual connection. Therefore, program mechanism 101 and the detection mechanism 100 can be attached to the same position of the user's skin while the infusion mechanism 102 is attached to the other position.

Another embodiment of the present invention is that the three are provided in different parts, thus being attached to different positions. Simultaneously, program mechanism 101, detection mechanism 100, and infusion mechanism 102 transmit wireless signals to realize the mutual connection.

Another embodiment of the present invention is that the three are provided in different parts, the detection mechanism 100 and the infusion mechanism 102 are attached to different locations of the user's skin, respectively, while the program mechanism 101 does not have to be attached to the skin, and control the detection mechanism 100 and the infusion mechanism 102 by means of a handheld or portable device. At this time, the program mechanism 101 transmits wireless signals to the detection mechanism 100 and the infusion mechanism 102, respectively, to achieve connection with each other.

The wireless connections described in the foregoing embodiments include, but are not limited to, radio frequency (RF) communications (e.g., radio frequency identification (RFID), Zigbee communication protocol, WiFi, infrared, wireless Universal Serial Bus (USB), Ultra Wide Band (UWB), Bluetooth^{®} communication protocol, and cellular communications, such as Code Division Multiple Access (CDMA) or the Global System for Mobile Communications (GSM).

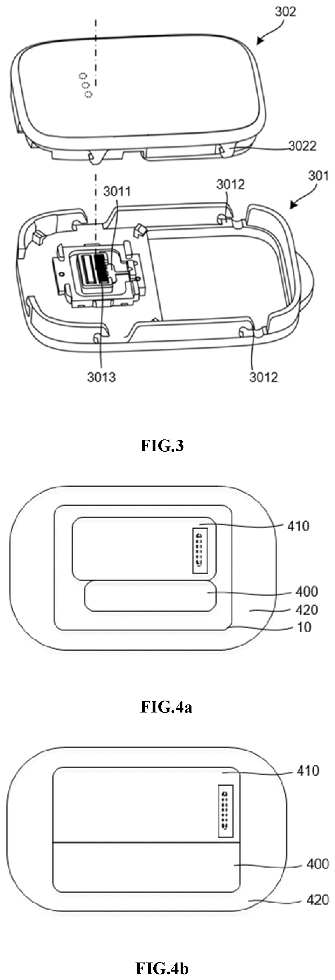

FIG.2 is a schematic diagram of the integrated continuous glucose monitoring device according to an embodiment of the present invention. FIG.3 is a schematic diagram of the split continuous glucose monitoring device according to an embodiment of the present invention.

The CGM comprising a sensor and a transmitter, mounted on the patient by means of an auxiliary mount, pierced subcutaneously. The sensor is used to collect analyte data in the human body and transmit the collected analyte content information. The transmitter is connected to the sensor, and it is used to receive the information on the blood glucose data, which is transmitted by the sensor that has been implanted under the skin, and convert the information into a wireless signal for outputting. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the CGM, or may also be set differently depending on the type of the CGM.

FIG.2 is a schematic diagram of the integrated continuous glucose monitoring device, i.e., the sensor and the transmitter have been integrated together prior to use, and is a single-use product, which is discarded after use, as shown in FIG. 2. The integrated continuous glucose monitoring device includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier may be provided on the outer housing of the CGM or on the outer packaging or within the CGM.

FIG.3 is a schematic diagram of the split continuous glucose monitoring device, i.e., the sensor and the transmitter are two different parts prior to use, packaged separately, and only integrated together when in use, as shown in FIG. 3. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the present invention, both the sensor and the transmitter of the split continuous glucose monitoring device are single-use products, which are discarded after use, and therefore the identifier may be provided on the housing or the outer packaging of the sensor or the transmitter. In yet another embodiment of the present invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

FIG.4a is a schematic diagram of the integrated insulin pump according to an embodiment of the present invention. FIG.4b is a schematic diagram of the split insulin pump according to an embodiment of the present invention.

In this embodiment of the invention, the insulin pump is a patch-type insulin pump, i.e., an insulin pump that does not comprise a long catheter, includes an infusion mechanism and a control mechanism, and being integrally affixed to the surface of the user's skin by an adhesive patch, and the medication is infused directly from the reservoir to the subcutaneous area along the infusion needle.

Each insulin pump has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the insulin pump, or may also be set differently depending on the type of the insulin pump.

FIG.4a is a schematic diagram of the integrated insulin pump, i.e., the infusion mechanism 410 and the control mechanism 400 of the insulin pump are provided inside the same housing 10, and both are connected by wires and are affixed to a location on the user's skin by means of an adhesive patch 420, which is disposed of in its entirety after a single use; an identifier may be provided on the outer housing of the insulin pump or on the outer packaging or inside the insulin pump.

FIG.4b is a schematic diagram of the split insulin pump, i.e., the infusion mechanism 410 and the control mechanism 400 of the insulin pump are provided in two different housings, both of which are connected by waterproof plugs or directly snap together and electrically connected as a whole, an identifier may be provided on the outer housing of the infusion mechanism and/or the control mechanism or on the outer packaging or inside the insulin pump.

In one embodiment of the present invention, both the infusion mechanism and the control mechanism of the split insulin pump are single-use products, which are discarded after use, and therefore the identifiers may be provided on the housing or on the outer packaging of the infusion mechanism and/or the control mechanism. In yet another embodiment of the present invention, only the infusion mechanism of the split insulin pump is a single-use product, while the control mechanism is a reusable product, and therefore, preferably, in this embodiment, the identifier is set on the housing of the control mechanism or on the outer packaging, which can reduce the frequency of binding of patient information and identifiers, and improve the patient experience. This is described in more detail below.

When the identifier is set on the housing of the control mechanism or on the outer packaging, it is set in a form that includes, but is not limited to, a QR code, a bar code, or an NFC tag.

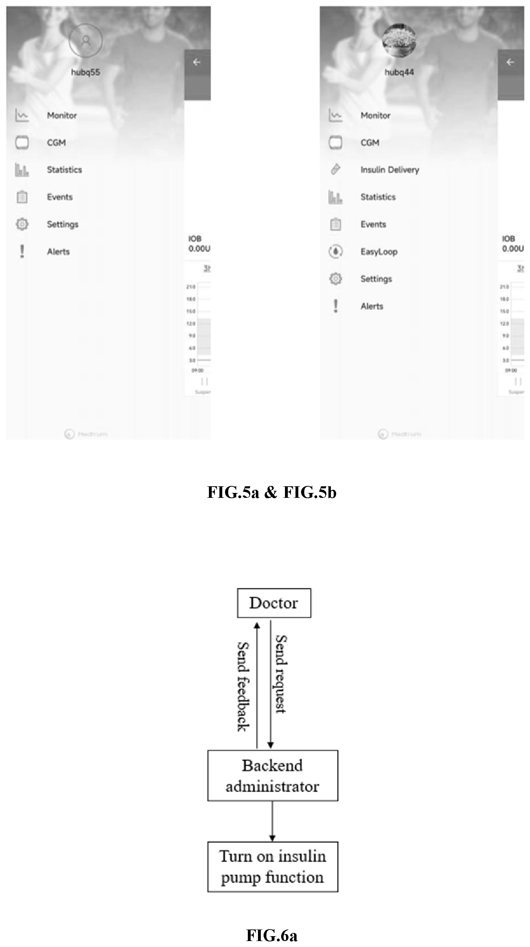

As mentioned earlier, considering the severity of illness in patients and the individual patient's physical health status, some patients may only need a CGM for continuous glucose monitoring, while others may need not only a CGM for continuous glucose monitoring, but also an insulin pump for drug infusion. When a doctor determines that a patient only needs to use a CGM for continuous glucose monitoring, and as the CGM involves only the monitoring of the patient's user's blood glucose, and the user's own use of the CGM does not pose a risk to the user's life and safety, the users may purchase the CGM by themselves. Before the CGM is installed on the surface of the user's skin, the user can search for and download the dedicated APP for controlling the CGM from the smartphone's app store, create a new account on the dedicated APP, and pair the patient's personal information with the information of the CGM to be worn, thus realizing the pairing and controlling of the smartphone and the CGM. The CGM and the insulin pump in the embodiment of the present invention are developed and produced by the same manufacturer, and thus can be controlled by the same dedicated APP in the smartphone, and since the insulin pump is not required to be used by all patients, only CGM-related content is involved in the default main screen of the dedicated APP, as shown in FIG. 5a. On the one hand, it can simplify the interface of the APP and provide the user with a good visual experience, and on the other hand, prevent the user from mis-operation on the insulin pump, which may affect the normal use of the CGM.

In one embodiment of the present invention, when a doctor determines that a patient needs to use an insulin pump for drug infusion, as shown in FIG. 6a, the doctor sends a request to the backend administrator to add the patient's account to a whitelist to allow the patient to use the insulin pump function, and the backend administrator receives the request to add a whitelist sent by the doctor and adds the patient's account to a list of whitelists, and at the same time sends the doctor a feedback that the addition of the whitelist has been completed, and furthermore the backend administrator turns on directly the insulin pump function on the app interface that the patient is using. At this time, the APP interface changes from FIG. 5a to FIG. 5b, and the interface of FIG. 5b adds two function keys related to insulin infusion, "Insulin Delivery" and "Easyloop", compared with the interface of FIG. 5a. In another embodiment of the present invention, the backend administrator does not directly turn on the insulin pump function of the APP interface used by the patient, but sends a security code to the patient's account, so that the patient can turn on the insulin pump function on the APP interface through the security code when needed or convenient.

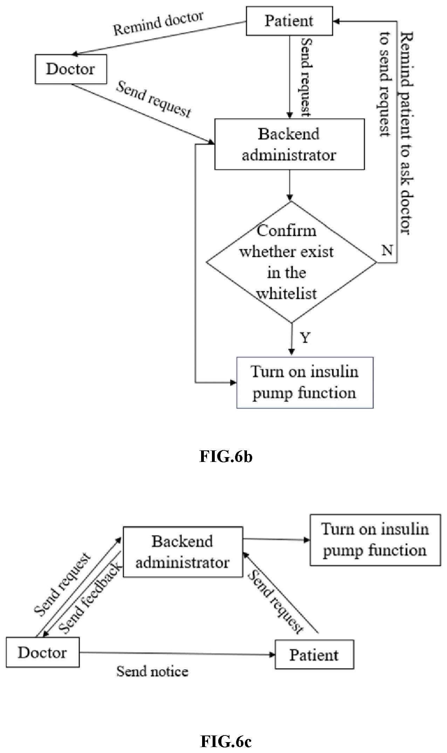

In another embodiment of the present invention, when the doctor determines that the patient needs to use the insulin pump for drug infusion, as shown in FIG. 6b, the patient can send an application to turn on the insulin pump function directly to the backend administrator, and the backend administrator receives the application to turn on the insulin pump function sent by the patient and verifies that whether the patient's account is in the whitelist or not, and if the patient's account is in the whitelist, the backend administrator turns on the insulin pump function in the APP interface that the patient is using and the APP interface changes from FIG. 5a to FIG. 5b; If the patient's account is not in the whitelist, a feedback message is sent to the patient's account to remind the patient to ask the doctor to send an add whitelist request to the backend administrator. After the doctor sends the whitelist application to the background administrator, the background administrator can directly turn on the insulin pump function of the APP interface used by the patient. If no message is received from the background administrator within a certain period of time, such as 1min, 2min, 5min, then another application for turning on the insulin pump function to the backend administrator can be send again, or ask the doctor to send the application for adding whitelist to the backend administrator. In another embodiment of the present invention, the backend administrator does not directly turn on the insulin pump function on the APP interface used by the patient, but instead sends a security code to the patient's account, which allows the patient to turn on the insulin pump function of the APP interface through the security code when needed or convenient.

In yet another embodiment of the present invention, when the doctor determines that the patient needs to use the insulin pump for drug infusion, as shown in FIG. 6c, the doctor sends a request to the backend administrator to add the patient account to the whitelist to allow the patient to use the insulin pump function, and the backend administrator receives the request sent by the doctor to add a whitelist and adds the patient account to the whitelist, and at the same time sends the doctor a feedback that the whitelist addition has been completed, and further the doctor notifies the patient that the patient can apply to use the insulin pump function. After receiving the notification from the doctor, the patient sends an application for turning on the insulin pump function to the backend administrator, and after the backend administrator receives the application for turning on the insulin pump function sent by the patient, the backend administrator directly turns on the insulin pump function on the APP interface used by the patient. In another embodiment of the present invention, after the backend administrator receives the application to turn on the insulin pump function sent by the patient, the backend administrator may also first verify whether the patient account exists in the whitelist or not, and if it is determined that the patient account is in the whitelist, the backend administrator turns on the insulin pump function on the APP interface used by the patient. In another embodiment of the present invention, the backend administrator does not directly turn on the insulin pump function on the APP interface used by the patient, but instead sends a security code to the patient's account, through which the patient can turn on the insulin pump function on the APP interface when needed or convenient.

When the doctor determines that the patient no longer needs to turn on the insulin pump function, the patient can turn off the insulin pump function on the APP by himself/herself, and the APP automatically sends a message to the backend administrator, who deletes the patient's account from the whitelist; the doctor and/or the patient can also send an application to the backend administrator to request to turn off the insulin pump function, and the backend administrator will turn off the insulin pump function on the APP and at the same time delete that patient account from the whitelist. When this patient needs to turn on the insulin pump function again, the patient account needs to be re-added to the whitelist by referring to one of method as shown in FIG. 6a- FIG.6c.

It should be noted that it is necessary to ensure that the insulin pump is properly mounted on the skin before turning on the insulin pump function of the APP, and to pair the patient's personal information with the information of the insulin pump, so as to realize the pairing and controlling of the smartphone and the insulin pump. The personal information of the patient includes name, age, gender, cell phone number, etc., and the information of the CGM and/or insulin pump worn includes the identifier information of the CGM and/or insulin pump. At the same time, the smartphone uploads the personal information of the patient and the identifier information of the CGM and/or the insulin pump to a remote server, which can store the information uploaded by the smartphone and verify whether the identifier information of the CGM and/or the insulin pump is valid, and if a certain identifier information already exists in the remote server, the remote server sends an alert to the smartphone to remind the user that the CGM or insulin pump has been used and needs to be replaced. When the CGM and/or the insulin pump is mounted on the skin of the patient and successfully activated, the CGM and/or the insulin pump starts to work, the transmitter of the CGM sends the monitored blood glucose information to the smartphone and further uploads it to the remote server, and the control mechanism of the insulin pump receives the insulin infusion information and controls the infusion mechanism to infuse the insulin, and at the same time sends the infusion status to the smartphone and further uploads it to the remote server.

It should be noted that the CGM and insulin pump in the embodiment of the present invention are developed and produced by the same manufacturer, and thus can be controlled by the same dedicated APP in the smartphone. Assuming that CGM or insulin pumps produced by other manufacturers also can be controlled by a dedicated APP in the smartphone, and the inconvenience to the user due to the use of different APPs for controlling the CGM and the insulin pump respectively can be avoided, and the user experience can be improved.

When the CGM and/or insulin pump worn by the patient needs to be replaced for reasons such as reaching the end of its life cycle or failing, the unique identifier information of the new CGM and/or insulin pump also needs to be updated by pairing with the patient's personal information via the smartphone and further uploaded to a remote server. The patient's personal information is entered manually, and the identifier information of the CGM and/or insulin pump may also be entered manually or by scanning a QR code, a barcode, or NFC tags on the housing or on the outer packaging of the CGM and/or the insulin pump.

When the CGM is split and the transmitter is reusable, the identifier of the CGM is set on the housing or packaging of the transmitter, so that the patient only needs to replace the sensor when replacing the CGM without replacing the transmitter, and the identifier of the CGM remains unchanged, and thus there is no need for updating the pairing of the identifier of the CGM with the personal information of the patient via a smartphone, and no need for uploading it to a remote server, and thus the number of operational steps can be reduced to improve the patient experience.

When the insulin pump is split and the control mechanism is reusable, the identifier of the insulin pump is set on the housing or packaging of the control mechanism, and the patient only needs to change the infusion mechanism when replacing the insulin pump without changing the control mechanism, and the identifier of the insulin pump remains unchanged, and thus there is no need for updating the pairing update of the identifier of the insulin pump with the personal information of the patient via the smartphone or in uploading it to a remote server, and thus the operational steps can be reduced and the patient experience can be improved.

The smartphone and the CGM and/or insulin pump, and the remote server communicate with each other via wireless communication, which may be via, for example, but not limited to, radio frequency (RF) communication (e.g., radio frequency identification (RFID), Zigbee communication protocols, WiFi, infrared, wireless Universal Serial Bus (USB), Ultra Wide Band (UWB), Bluetooth^{®} communication protocols, and cellular communication, such as Code Division Multiple Access (CDMA) or Global System for Mobile Communications (GSM). communications, such as Code Division Multiple Access (CDMA) or Global System for Mobile Communications (GSM). Preferably, the smartphone and the remote server communicate with each other via WiFi and/or cellular communication, and the smartphone and the CGM and/or insulin pump communicate with each other via Bluetooth^{®} communication protocol.

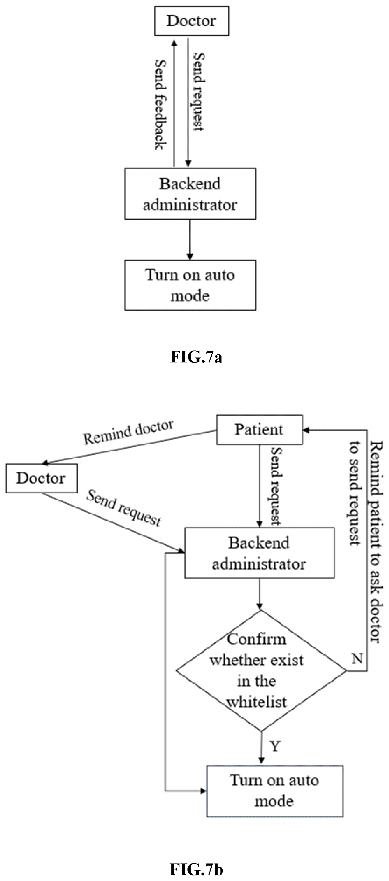

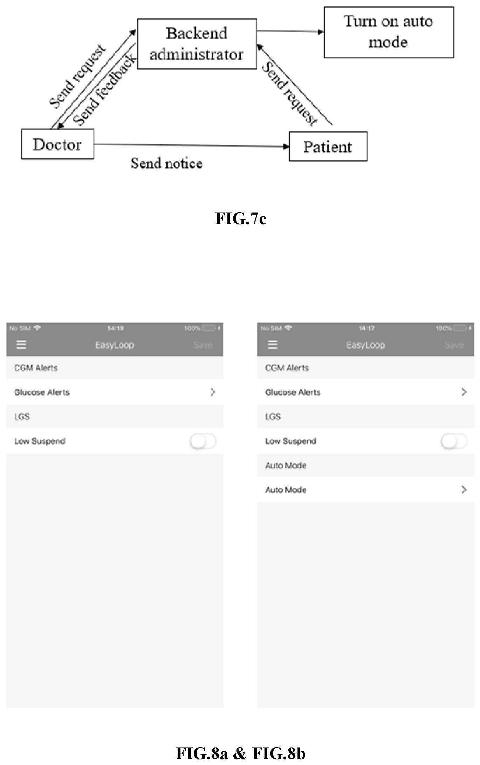

When the doctor determines that the patient can turn on the automatic mode, i.e. after the APP reads the current blood glucose value monitored by the CGM and the insulin information infused by the insulin pump, it can predict the future trend of blood glucose and control the infusion of the insulin pump based on the predicted trend of blood glucose, including increasing, decreasing, or stopping the infusion of insulin, in order to affect the blood glucose values, and form an automated closed-loop control cycle. As shown in FIG. 7a, the doctor sends a request to the backend administrator to add the patient account to the whitelist to allow the patient to use the auto mode function, and the backend administrator receives the add whitelist request sent by the doctor and adds the patient account to the whitelist, and at the same time sends the feedback to the doctor that the whitelist has been added, and furthermore directly turns on the auto mode function on the APP interface used by the patient. At this time, the APP interface changes from FIG. 8a to FIG. 8b, and the interface of FIG. 8b has added "Auto Mode", i.e., a function key related to the auto mode, compared with the interface of FIG. 8a. In another embodiment of the present invention, the backend administrator does not directly turn on the Auto Mode function on the APP interface used by the patient, but sends a security code to the patient's account, so that the patient can turn on the Auto Mode function on the APP interface through the security code when needed or convenient.

In another embodiment of the present invention, when the doctor determines that the patient needs to use the insulin pump for drug infusion, as shown in FIG. 7b, the patient can send a request to turn on the auto mode function directly to the backend administrator, and the backend administrator, upon receipt of the request sent by the patient to turn on the auto mode function, verifies whether the patient's account is in the whitelist or not, and if the patient's account is in the whitelist, the backend administrator turns on the auto mode function on the APP interface that is used by the patient, and the APP interface changes from FIG. 8a to FIG. 8b. If the patient's account is not in the whitelist, a feedback message is sent to the patient's account to remind the patient to ask the doctor to send an add whitelist request to the background administrator. After the doctor sends the whitelist application to the backend administrator, the backend administrator can directly turn on the auto mode function on the APP interface used by the patient. If no message is received from the backend administrator within a certain period of time, such as 1min, 2min, 5min, another application for turning on the insulin pump function to the backend administrator can be sent again, or ask the doctor to send the application for adding whitelist to the backend administrator. In another embodiment of the present invention, the backend administrator does not directly turn on the auto mode function on the APP interface used by the patient, but instead sends a security code to the patient's account, through which the patient can turn on the auto mode function on the APP interface when needed or convenient.

In yet another embodiment of the present invention, when the doctor determines that the patient needs to use the insulin pump for drug infusion, as shown in FIG. 7c, the doctor sends a request to the backend administrator to add the patient account to the whitelist to allow the patient to use the auto mode function, and the backend administrator receives the request to add a whitelist sent by the doctor and adds the patient account to the whitelist, and at the same time sends a feedback to the doctor that the whitelist has been added, and furthermore the doctor notifies the patient that he/she can request to use the auto mode function. After receiving the notification from the doctor, the patient sends an application for turning on the auto mode function to the backend administrator, and after the backend administrator receives the application for turning on the auto mode function sent by the patient, the backend administrator directly turns on the auto mode function on the APP interface used by the patient. In another embodiment of the present invention, after the backend administrator receives the application for turning on the auto mode function sent by the patient, the backend administrator may also first verify whether the patient account exists in the whitelist or not, and if it is determined that the patient account is in the whitelist, the backend administrator turns on the auto mode function on the APP interface used by the patient. In another embodiment of the present invention, the backend administrator does not directly turn on the auto mode function on the APP interface used by the patient, but instead sends a security code to the patient's account, through which the patient can turn on the auto mode function on the APP interface when needed or convenient.

When the doctor determines that the patient no longer needs to turn on the auto mode function, the patient can turn off the auto mode function on the APP by themselves, and the APP automatically sends a message to the backend administrator, who deletes the patient account from the whitelist. Or the doctor and/or the patient can also send a request to the backend administrator to turn off the auto mode function, and the backend administrator turns off the auto mode function on the APP, and at the same time deletes the patient account from the whitelist. When this patient needs to turn on the auto mode function again, the patient account needs to be re-added to the whitelist by referring to one of the methods as shown in FIG. 7a-FIG. 7c.

In summary, the present invention discloses a control system for blood glucose monitoring and management. The first working mode of the control system is used to control the blood glucose monitoring device to perform real-time blood glucose monitoring, and the system enters the second working mode based on a trigger. The second working mode at least is used to control the blood glucose monitoring device to perform real-time blood glucose monitoring and control the insulin infusion device to perform insulin infusion. By a trigger, the control system transitions from the first working mode of real-time blood glucose monitoring by controlling the blood glucose monitoring device to the second working mode of at least real-time blood glucose monitoring by controlling the blood glucose monitoring device and insulin infusion by controlling the insulin infusion device, preventing patients from accidentally entering unnecessary or unacceptable working mode and improving the safety of blood glucose monitoring and management for patients.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. A control system for blood glucose monitoring and management, comprising a processor, and a memory storing programming instructions executable by the processor to cause the control system to:
operate in at least a first working mode, configured to control a blood glucose monitoring device to perform real-time blood glucose monitoring; wherein:
upon a trigger, transition to a second working mode configured to control the blood glucose monitoring device to perform real-time blood glucose monitoring and to control an insulin infusion device to perform insulin infusion.

2. The control system for blood glucose monitoring and management of claim 1, wherein,
the trigger at least includes a patient account that needs to enter the second working mode is included in a whitelist of a backend administrator.

3. The control system for blood glucose monitoring and management of claim 2, wherein,
the whitelist is generated by a doctor sends a request to add a whitelist to the backend administrator.

4. The control system for blood glucose monitoring and management of claim 3, wherein,
the backend administrator directly controls the control system to enter the second working mode after adding the whitelist.

5. The control system for blood glucose monitoring and management of claim 3, wherein,
the backend administrator sends a security code to the patient account after adding the whitelist, and the patient controls the control system to enter the second working mode via the security code.

6. The control system for blood glucose monitoring and management of claim 3, wherein,
a patient alerts the doctor to send the request to the backend management.

7. The control system for blood glucose monitoring and management of claim 3, wherein,
the backend administrator sends feedback to the doctor after receiving the request, the doctor notices a patient to send another request to the backend administrator upon receipt of the feedback.

8. The control system for blood glucose monitoring and management of claim 7, wherein,
the backend administrator directly controls the control system to enter the second working mode after receiving the request.

9. The control system for blood glucose monitoring and management of claim 7, wherein,
the backend administrator sends a security code to the patient account after receiving the request, and the patient controls the control system to enter the second working mode via the security code.

10. The control system for blood glucose monitoring and management of claim 1, wherein,
the second working mode further includes an auto mode.

11. The control system for blood glucose monitoring and management of claim 10, wherein,
the trigger for the auto mode at least includes a patient account that needs to enter the auto mode is included in a whitelist of a backend administrator.

12. The control system for blood glucose monitoring and management of claim 11, wherein,
the whitelist is generated by a doctor sends a request to add a whitelist to the backend administrator.

13. The control system for blood glucose monitoring and management of claim 12, wherein,
the backend administrator directly controls the control system to enter the auto mode after adding the whitelist.

14. The control system for blood glucose monitoring and management of claim 12, wherein,
the backend administrator sends a security code to the patient account after adding the whitelist, and the patient controls the control system to enter the auto mode via the security code.

15. The control system for blood glucose monitoring and management of claim 12, wherein,
a patient alerts the doctor to send the request to the backend management.

16. The control system for blood glucose monitoring and management of claim 12, wherein,
the backend administrator sends feedback to the doctor after receiving the request, the doctor notices a patient to send another request to the backend administrator upon receipt of the feedback.

17. The control system for blood glucose monitoring and management of claim 16, wherein,
the backend administrator directly controls the control system to enter the auto mode after receiving the request.

18. The control system for blood glucose monitoring and management of claim 17, wherein,
the backend administrator sends a security code to the patient account after receiving the request, and the patient controls the control system to enter the auto mode via the security code.

19. The control system for blood glucose monitoring and management of claim 1, wherein,
the control system is a smartphone.

20. The control system for blood glucose monitoring and management of claim 19, wherein,
the control system is an APP in the smartphone.

21. The control system for blood glucose monitoring and management of claim 20, wherein,
after the control system enters the second working mode, insulin infusion related functions displayed on a main interface of the APP.

22. The control system for blood glucose monitoring and management of claim 2, wherein,
the control system exits the second working mode when the patient account is removed from the whitelist.
